(19) Europäisches Patentamt
European Patent Office
Office européen des brevets

(11) Veröffentlichungsnummer: **0 223 735 B1**

# (12) EUROPÄISCHE PATENTSCHRIFT

(45) Veröffentlichungstag der Patentschrift: **08.11.89**

(51) Int. Cl.[4]: **C 07 F 7/18**, C 07 C 47/14, C 07 C 47/353

(21) Anmeldenummer: **86810453.0**

(22) Anmeldetag: **15.10.86**

(54) **Verfahren zur Herstellung von Halbaminalen, Halbaminale und deren Verwendung.**

(30) Priorität: **21.10.85 CH 4515/85**

(43) Veröffentlichungstag der Anmeldung: **27.05.87 Patentblatt 87/22**

(45) Bekanntmachung des Hinweises auf die Patenterteilung: **08.11.89 Patentblatt 89/45**

(84) Benannte Vertragsstaaten: **AT BE CH DE FR GB IT LI NL**

(56) Entgegenhaltungen:
**EP-A- 0 113 467**
**EP-A- 0 155 885**
**DE-B- 2 163 752**
**DE-B- 2 613 003**
**GB-A- 1 091 549**
**US-A- 2 852 569**
**US-A- 2 870 213**
**US-A- 3 038 941**
**US-A- 3 344 193**

**HOUBEN-WEYL: "Methoden der organischen Chemie", Band E 3, 1983, Seiten 383-386, Georg Thieme Verlag, Stuttgart**

(73) Patentinhaber: **CIBA-GEIGY AG, Klybeckstrasse 141, CH-4002 Basel (CH)**

(72) Erfinder: **Lang, Robert Werner, Dr., Hagenbachweg 10, CH-4133 Pratteln (CH)**

**EP 0 223 735 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von Halbaminalen durch Umsetzung von Dimethylformamid, einem Monohalogensilan und einem organischen Polyhalogenid in Gegenwart von Zink, wobei das organische Polyhalogenid und Zink auch als organischer Zinkhalogenid-Komplex eingesetzt werden können, die neuen Halbaminale und die Verwendung der Halbaminale zur Herstellung von polyhalogenierten Aldehyden.

Aus Tetrahedron Letters, S. 3867-3870 (1968) ist 1,1,1-Trichlor-2-dimethylamino-2-trimethylsilyloxyethan bekannt. Die Formylierung von metallorganischen Verbindungen auf der Basis von Lithium und Magnesium mit Dimethylformamid (DMF) ist z.B. in Tetrahedron Letters, S. 1143 (1983) beschrieben. Die Umsetzung von polyhalogenierten metallorganischen Verbindungen mit DMF in Gegenwart eines Monohalogensilans zu silylierten Halbaminalen ist nicht bekannt.

Ein Gegenstand vorliegender Erfindung ist ein Verfahren zur Herstellung von Halbaminalen der Formel I

```
   R¹
   |
R²-SiO-CH-N(CH₃)₂        (I),
   |   |
   R³  R
```

worin R unsubstituiertes oder mit $-NO_2$, -CN, -COR', -COOR', $-CON(R')_2$, $C_1$-$C_{12}$-Alkyl, $C_1$-$C_{12}$-Alkoxy, $C_1$-$C_{12}$-Alkylthio, $C_1$-$C_{12}$-Sulfoxyl, $C_1$-$C_{12}$-Sulfonyl, Phenyl, Benzyl, $C_3$-$C_{18}$-Trialkylsilyl oder -silyloxy und $C_1$-$C_{18}$-Mono- oder Dialkylamino, substituiertes polyhalogeniertes Alkyl, Cycloalkyl oder Aralkyl mit jeweils mindestens einem F-Atom bedeutet, wobei R' ein Wasserstoffatom, $C_1$-$C_{18}$-Alkyl, $C_4$-$C_8$-Cycloalkyl, Phenyl oder Benzyl bedeutet, oder die Arylgruppe von R als Aralkyl auch mit -OH substituiert sein kann und $R^1$, $R^2$ und $R^3$ unabhängig voneinander ein Wasserstoffatom, Alkyl, Cycloalkyl oder Aryl darstellen, das dadurch gekennzeichnet ist, dass man

a) mindestens äquimolare Mengen Dimethylformamid, eines Silans der Formel II

$$R^1R^2R^3SiX \qquad (II),$$

worin X C1 oder Br ist, und $R^1$, $R^2$, $R^3$ die zuvor angegebene Bedeutung haben, und einer Verbindung der Formel III

$$RY \qquad (III),$$

worin R die zuvor angegebene Bedeutung hat und Y für Cl, Br oder J steht, in Gegenwart von Zink miteinander umsetzt, oder

b) mindestens äquimolare Mengen Dimethylformamid, eines Silans der Formel II und einer Zinkverbindung der Formel IV

$$RZnY \cdot yL \qquad (IV),$$

worin R und Y die zuvor angegebene Bedeutung haben, L einen Liganden bedeutet und y 1 oder 2 ist, miteinander umsetzt.

In den Verbindungen der Formeln I und II stellen $R^1$, $R^2$ und $R^3$ als Alkyl bevorzugt lineares oder verzweigtes $C_1$-$C_{18}$-, besonders $C_1$-$C_{12}$- und insbesondere $C_1$-$C_8$-Alkyl, als Cycloalkyl bevorzugt Cyclopentyl oder Cyclohexyl und als Aryl bevorzugt Phenyl dar. Besonders bevorzugt sind $R^1$, $R^2$ und $R^3$ gleiche oder verschiedene Alkylreste mit insbesondere 1-8 C-Atomen.

Beispiele für $R^1$, $R^2$ und $R^3$ sind: Methyl, Ethyl, n-Propyl, i-Propyl, n-, i- oder t-Butyl, Pent-l-yl, 1-, 2- oder 3-Hexyl, 1,2,2-Trimethyleth-l-yl, 1,1,2,2-Tetramethyleth-l-yl (Thexyl), Heptyl, Octyl, Decyl, Dodecyl, Octadecyl, Cyclohexyl, Methylcyclohexyl, Phenyl und Methylphenyl.

Beispiele für die $R^1R^2R^3Si$-Gruppe sind: Silyl, Methyl-, Dimethyl-, Trimethyl-, Ethyl-, Methylethyl-, Diethyl-, Triethyl-, Dimethylethyl-, Propyl-, i-Propyl-, i-Propyldimethyl-, Tri-n-Propyl-, Tri-n-Butyl-, n-Butyldimethyl-, n-Butyldiethyl-, t-Butyldimethyl-, Tri-n-Pentyl-, n-Pentyldimethyl-, (1,2,2-trimethyleth-l-yl)dimethyl-, (1,1,2,2-Tetramethylethyl)dimethyl-, Tri-n-Octyl- und n-Octyldimethylsilyl.

Der Rest R in den Formeln I, III und IV ist mit F polyhalogeniert wobei das Fluor teilweise durch Cl und/oder Br substituiert sein kann. Der Rest R kann substituiert oder unsubstituiert sein. Für den Fall der Substitution enthält R bevorzugt 1 bis 3, besonders 1 oder 2 und insbesondere 1 Substituenten.

R' als Alkyl kann linear oder verzweigt sein, das bevorzugt 1 bis 12 C-Atome enthält.

Beispiele für Substituenten sind neben $-NO_2$ und -CN auch Carbomethyl, Carboethyl, Carbophenyl, Carboxyl, Carbomethoxy, Carboethoxy, Carbopropoxy, Carbobutoxy, Carbamino, Methylcarbamino, Dimethylcarbamino, Ethylcarbamino, Methylethylcarbamino, Methyl, Ethyl, n-Propyl, i-Propyl, n-Butyl, i-Butyl, t-Butyl, Methoxy, Ethoxy, Propoxy, Methylthio, Trimethylsilyl, Trimethylsilyloxy, Methylamino, Dimethylamino, Methylethylamino.

R kann als polyhalogeniertes Alkyl linear oder verzweigt sein und vorzugsweise 1 bis 18, besonders 1-12 C-Atome enthalten. Als fluoriertes Cycloalkyl enthält R vorzugsweise 3-8 C-Atome im Ring. R als fluoriertes Aralkyl ist bevorzugt α-Fluor-α-chlor- oder α,α-Difluorbenzyl, Mono-, Di-, Tri-, Tetra- oder Pentafluorbenzyl, -α-fluorbenzyl oder -α,α-difluorbenzyl. R ist bevorzugt mit F und Cl polyhalogeniert.

Beispiele für R sind: $CF_3$, $CF_2Cl$, $CFCl_2$, $CF_2CF_3$, $CF_2ClCF_2$, $CCl_3CF_2$, $CF_3CCl_2$, $CF_3CFCl$, $CFCl_2CF_2$, $CFCl_2CCl_2$, $C_3F_9$, $CF_3CF_2CCl_2$, $C_nF_{2n+1}$ (Perfluoralkylreste) mit n = 4-18, bevorzugt 4-12, $C_6F_5CF_2$, $C_2H_5OOCCF_2$, $C_6H_5COCF_2$.

Die Verbindungen der Formel II sind bekannt oder können nach bekannten Verfahren hergestellt werden. Verbindungen der Formel II, in denen einer der Reste $R^1$, $R^2$ oder $R^3$ ein disubstituiertes α-C-Atom enthält (z.B. die Thexylgruppe), können durch die Umsetzung von Monohalogendialkylsilanen mit tetrasubstituierten Ethylenen in Gegenwart von $AlBr_3$ oder $AlCl_3$ hergestellt werden. X in Formel II ist bevorzugt Cl.

Die Verbindungen der Formel III sind bekannt oder können nach bekannten Verfahren hergestellt werden. Sofern Y Cl bedeutet, ist es zweckmässig, zur Reaktionsbeschleunigung katalytische Mengen Jod zuzufügen.

Die Zinkverbindungen der Formel IV sind bekannt (vgl. US-PS-3 290 333) oder können nach analogen Verfahren hergestellt werden. Ferner können Zinkverbindungen der Formel IV auch so erhalten werden, dass man einen leichter gebundenen Liganden wie z.B. Ether, durch Säureamide wie z.B. DMF, verdrängt. Sofern Zinkverbindungen der Formel IV instabil sind, ist es zweckmässig, diese Zinkverbindungen in Lösung einzusetzen und gegebenenfalls erst vor der Durchführung der Reaktion herzustellen. Als Lösungsmittel verwendet man hierbei bevorzugt solche, die dem Liganden L entsprechen.

L in Formel II entspricht bevorzugt einem Hetroatome, wie z.B. N, S und O enthaltenden aprotischen Lösungsmittel. Insbesondere ist L ausgewählt aus der Gruppe der Ether, der Carbonsäureester und Lactone, der Sulfoxide und Sulfone und der N-substituierten Säureamide und der Lactame.

Geeignete Lösungsmittel sind zum Beispiel: Ether wie Diethylether, Dibutylether, Tetrahydrofuran, Dioxan, Dimethylethylenglykol, Dimethyldiethylenglykol, Diethyldiethylenglykol, Dibutyldiethylenglykol, Dimethyltriethylenglykol, Carbonsäureester und Lactone wie Propylencarbonat, Essigsäureethylester, Propionsäuremethylester, Benzoesäureethylester, Ethylglykolacetat, 2-Methoxyethylacetat, γ-Butyrolacton, γ-Valerolacton und Mevalolacton, Sulfoxide, wie Dimethylsulfoxid, Tetramethylensulfoxid, Sulfone wie Dimethylsulfon, Diethylsulfon, Trimethylensulfon, Tetramethylensulfon, und besonders Säureamide wie z.B. Dimethylformamid, Dimethylacetamid, N-Methylpyrrolidon und Hexamethylphosphorsäuretriamid.

Das Zink wird vorteilhaft nach Fieser und Fieser, Reagents for Organic Synthesis, Vol. I, John Wiley, N.Y., S. 1276 (1967) aktiviert.

Das erfindungsgemässe Verfahren kann unter Einwirkung von Ultraschall durchgeführt werden.

Die Reaktion a) wird bevorzugt bei Temperaturen von −30 °C bis 140 °C, besonders 0 bis 80 °C durchgeführt. Die Reaktion b) wird vorteilhaft bei Temperaturen von 20°C bis 200°C, besonders 50 bis 150 °C durchgeführt.

Die Reaktanden werden in mindestens äquimolaren Mengen eingesetzt, wobei das Halogensilan der Formel II auch in geringem Überschuss eingesetzt werden kann. Bei Reaktion a) kann DMF in wesentlich höherer Menge eingesetzt werden und gleichzeitig als Lösungsmittel dienen. Man kann aber auch eine äquimolare Menge DMF einsetzen, wobei sich dann die Mitverwendung eines inerten Lösungsmittels empfiehlt. Geeignet sind polare aprotische Lösungsmittel, wie sie z.B. zuvor beschrieben worden sind. Die Reihenfolge der Zugabe der Reaktanden ist an sich beliebig. Es ist lediglich darauf zu achten, dass der letzte Reaktand wegen der exothermen Reaktion langsam zugegeben wird, damit die Reaktionstemperatur nicht zu stark ansteigt. In einer bevorzugten Ausführungsform werden entweder Zink oder die Verbindung der Formel III zuletzt in die Mischung der übrigen Reaktanden eingetragen.

Bei der Reaktion b) kann die Zinkverbindung der Formel IV in einem inerten Lösungsmittel gelöst und danach zuerst DMF und dann das Halogensilan der Formel II oder umgekehrt zugesetzt werden. Vorteilhaft wird die Zinkverbindung der Formel IV direkt vor der Reaktion durch Umsetzung von Zink und einer Verbindung der Formel III in Gegenwart eines Lösungsmittels erzeugt, das dem Liganden L entspricht. In einer bevorzugten Ausführungsform entspricht L in Formel IV Dimethylformamid und y = 2, und die Reaktion b) wird vorteilhaft in Gegenwart eines inerten Lösungsmittels, besonders DMF, durchgeführt.

Das erfindungsgemässe Verfahren wird vorteilhaft unter Schutzgas (Argon oder Stickstoff) und unter Feuchtigkeitsausschluss durchgeführt.

Die Isolierung der Halbaminale der Formel I erfolgt in üblicher Weise. Die Reaktionsmischung kann z.B. mit einem Lösungsmittel wie Kohlenwasserstoffen (Pentan, Hexan, Cyclohexan, Petrolether) extrahiert werden. Der Extrakt wird dann gewaschen und getrocknet und das Lösungsmittel abdestilliert.

Mit dem erfindungsgemässen Verfahren werden erstmals polyhalogenierte, mindestens ein F-Atom enthaltende Halbaminale allgemein zugänglich. Ein weiterer Gegenstand vorliegender Erfindung sind Halbaminale der Formel I

$$\begin{array}{l} \quad R^1 \\ \quad | \\ R^2\text{-SiO-CH-N}(CH_3)_2 \\ \quad |\quad\;\; | \\ \quad R^3 \;\; R \end{array} \qquad (I),$$

worin R unsubstituiertes oder substituiertes polyhalogeniertes Alkyl, Cycloalkyl oder Aralkyl mit mindestens einem F-Atom bedeutet und $R_1$, $R_2$ und $R_3$ unabhängig voneinander ein Wasserstoffatom, Alkyl, Cycloalkyl oder Aryl darstellen. Für R und $R^1$-$R^3$ gelten die zuvor beschriebenen Bevorzugungen.

Die Halbaminale der Formel I sind wertvolle Zwischenprodukte zur Herstellung von halogenhaltigen pharmazeutischen und agrochemischen Wirkstoffen. Sie sind stabile Formen von polyhalogenierten Aldehyden.

Aldehyde der Formel R-CHO können z.B. durch Hydrolyse der Halbaminale mit starken Säuren wie $H_2SO_4$ hergestellt werden, vgl. Houben-Weyl, Band E 3: Aldehyde, S. 685ff. (1983).

Ein weiterer Gegenstand der Erfindung ist die Verwendung von Halbaminalen der Formel I zur Herstellung von Aldehyden der Formel R-CHO, durch Hydrolyse mit Mineralsäuren.

Die polyhalogenierten Aldehyde sind wertvolle Monomere für die kationische Polymerisation zur Herstellung von PTFE-ähnlichen Polymeren, vgl. Pure & Appl. Chem. *48*, S. 275 (1976).

Die nachfolgenden Beispiele erläutern die Erfindung näher. DMF ist Dimethylformamid. Als Schutzgas wird Stickstoff oder Argon verwendet.

*Beispiel 1*

*1,1,1-Trifluor-2-dimethylamino-2-trimethylsilyloxyethan*

In einem 100 ml Dreihalsrundkolben werden unter Schutzgas 50 ml DMF mit 3,3 g (50 mMol) Zinkstaub (aktiviert nach Fieser & Fieser) und 6,0 g (55 mMol) Trimethylchlorsilan versetzt und auf 0°C gekühlt. Über eine Glasfritte werden 7,3 g (40 mMol) Trifluorjodmethan unter kräftigem Rühren so eingeleitet, dass die Reaktionstemperatur der stark exothermen Reaktion 10°C nicht übersteigt. Nach 2 Stunden Ausrühren bei Raumtemperatur wird die gelbe Reaktionslösung mit Pentan extrahiert, die Pentanphase mit $H_2O$ gewaschen, über $Na_2SO_4$ getrocknet und anschliessend am Rotationsverdampfer bei Raumtemperatur (RT) eingeengt. Das gewünschte Halbaminal fällt dabei in 56% Ausbeute (4,8 g) und hoher Reinheit als gelbes Öl an. $^1$H-NMR (250 MHz, $CDCl_3$): 0,16 ppm, s, 9H, 3 $CH_3$-Si; 2,36 ppm, s, 6H, 2 $CH_3$-N; 4,46 ppm, qa, JHF = 5,5 Hz, 1H-C(2).

*Beispiel 2*

*1,1,1,2,2-Pentafluor-3-dimethylamino-3-thexyldimethylsilyloxypropan*

In einem 100 ml Dreihalsrundkolben werden unter Schutzgas 50 ml DMF mit 3,3 g (50 mMol) Zinkstaub und 9,8 g (55 mMol) Thexyldimethylchlorsilan versetzt und auf 0°C gekühlt. Über eine Glasfritte werden 13,5 g (55 mMol) Pentafluorethyljodid unter kräftigem Rühren so eingeleitet, dass die Reaktionstemperatur der exothermen Reaktion 10°C nicht übersteigt. Nach 2 Stunden Ausrühren bei RT wird analog Beispiel 1 aufgearbeitet und das Rohprodukt am Hochvakuum destilliert. Das gewünschte Halbaminal fällt dabei als farbloses Öl in 62% an.

Sdp. 50°C/0,013 mbar. $^1$H-NMR (300 MHz, $CDCl_3$): 2,40 ppm, s, 6H, 2 $CH_3$-N; 4,54 ppm, d x d, JFH = 10,0 Hz, 1H-C(3).

*Beispiel 3*

*1,1-Dichlor-1-fluor-2-dimethylamino-2-thexyldimethylsilyloxyethan*

In einem 100 ml Bombenrohr werden unter Schutzgas 50 ml DMF mit 3,3 g (50 mMol) Zinkstaub und 9,8 g (55 mMol) Thexyldimethylchlorsilan vorgelegt. Bei 0°C werden 7,6 g (55 mMol) Trichlorfluormethan sowie eine katalytische Menge Jod zugegeben. Nach 12 Stunden bei 70°C wird analog Beispiel 1 aufgearbeitet und das Rohprodukt am Hochvakuum destilliert. Das gewünschte Halbaminal fällt dabei als farbloses Öl in 43% Ausbeute (6,8 g) an.

Sdp. 80°C/0,013 mbar: $^1$H-NMR (60 MHz, $CDCl_3$): 2,44 ppm 2 s,
$\Delta\delta$ = 2 Hz, 6H, 2 $CH_3$-N; 4,40 ppm, d, JHF = 6,5 Hz, 1H-C(2).

*Beispiel 4*

*1,1,1-Trifluor-2,2-dichlor-3-dimethylamino-3-trimethylsilyloxypropan*

In einem 2,5 l Sulfierkolben werden unter Schutzgas 1000 ml DMF mit 65,5 g (1,0 Mol) Zinkstaub versetzt. Bei 0-10°C wird ein Gemisch von 187,5 g (1,0 Mol) 1,1,1-Trifluortrichlorethan und 130,2 g (1,2 Mol) Trimethylchlorsilan unter kräftigem Rühren zugetropft, wobei in einer exothermen Reaktion das Zink langsam in Lösung geht. Nach 2 Stunden Ausrühren bei RT wird analog Beispiel 1 aufgearbeitet. Das gewünschte Halbaminal fällt dabei in 62% Ausbeute (182,5 g) und hoher Reinheit als gelbes Öl an.

$^1$H-NMR (250 MHz, $CDCl_3$): 0,21 ppm, s, 9H, 3 $CH_3$-Si; 2,49 ppm, s, 6H, 2 $CH_3$-N; 4,71, s, 1H-C(3).

*Beispiel 5*

*1,1,1-Trifluor-2,2-dichlor-3-dimethylamino-3-tert.butyldimethylsilyloxypropan*

In einem 100 ml Dreihalsrundkolben werden unter Schutzgas 50 ml DMF mit 2,2 g (33 mMol) Zinkstaub und 4,5 g (30 mMol) tert.Butyldimethylchlorsilan versetzt. Bei 0-10°C werden 5,6 g (30 mMol) 1,1,1-Trifluortrichlorethan zugetropft und nach Abklingen der exothermen Reaktion während 2 Stunden bei RT belassen. Nach analoger Aufarbeitung wie Beispiel 1 fällt das gewünschte Halbaminal als gelbes Öl in 42% Ausbeute (4,3 g) an.

Sdp. 120°C/26 mbar; $^1$H-NMR (250 MHz, $CDCl_3$): 2,51 ppm, s, 6H 2 $CH_3$-N; 4,70 ppm, s, 1H-C(3).

*Beispiel 6*

1,1,1-Trifluor-2,2-dichlor-3-dimethylamino-3-thexyldimethylsilyloxypropan

*Methode A:*

In einem 250 ml Dreihalsrundkolben werden unter Schutzgas 100 ml DMF mit 6,8 g (0,104 Mol) Zinkstaub und 17,8 g (0,10 Mol) Thexyldimethylchlorsilan versetzt. Bei 0-10°C werden 20,7 g (0,11 Mol) 1,1,1-Trifluortrichlorethan zugetropft und nach Abklingen der exothermen Reaktion während 2 Stunden bei RT belassen. Nach analoger Aufarbeitung wie in Beispiel 1 fällt das gewünschte Halbaminal als hellgelbes Öl in 70% Ausbeute (25,6 g) an.

Sdp. 70°C/0,013 mbar; $^1$H-NMR (250 MHz, $CDCl_3$): 2,53 ppm, s, 6H, 2 $CH_3$-N; 4,72 ppm, s, 1H-C(3).

*Methode B:*

In einem 500 ml Dreihalsrundkolben werden unter Schutzgas 57,2 g (0,175 Mol) 2,2,2-Trifluordichlorethylzinkchlorid x2 Diethylether langsam und unter guter Kühlung mit 300 ml DMF versetzt. Nach Abklingen der exothermen Reaktion gibt man 34,2 g (0,191 Mol) Thexyldimethylchlorsilan zu und erhitzt während 2 Stunden auf 80°C. Die Aufarbeitung geschieht wie in Beispiel 1; Ausbeute 67% (43,1 g).

*Beispiel 7*

*1,1-Difluor-1-ethoxycarbonyl-2-dimethylamino-2-thexyldimethylsilyloxyethan*

In einem 250 ml Dreihalsrundkolben werden unter Schutzgas 120 ml DMF 4,32 g (66 mMol) Zinkstaub und 13,4 g (75 mMol) Thexyldimethylchlorsilan versetzt. Bei RT werden 9,51 g (60 mMol) Chlordifluoressigsäure zugetropft und anschliessend während 4 Stunden auf 80°C erhitzt. Nach analoger Aufarbeitung wie in Beispiel 1 fällt das gewünschte Halbaminal als hellgelbes Öl in 84% Ausbeute (17,0 g) an.

Sdp. 120°C/0,013 mbar; $^1$H-NMR (250 MHz, $CDCl_3$): 2,36 ppm, s, 6H, 2 $CH_3$-N; 4,59 ppm, d x d, JHF = 13,5 Hz, 1 H-C(2).

*Beispiel 8*

*1,1,1-Trifluor-2-dimethylamino-2-thexyldimethylsilyloxyethan*

Analoges Vorgehen wie in Beispiel 2 mit Trifluormethyljodid anstelle von Pentafluorethyljodid liefert das gewünschte Halbaminal als farbloses Öl in 74% Ausbeute.

Sdp. 60°C/0,013 mbar; $^1$H-NMR (250 MHz, $CDCl_3$): 2,40 ppm, s, 6H, 2 $CH_3$-N; 4,46 ppm, qa, JHF = 5,5 Hz, 1 H-C(2).

*Beispiel 9*

*1,1,1,2,2,3,3,4,4,5,5,6,6,7,7-Pentadecafluor--8-dimethylamino-8-thexyldimethylsilyloxyoctan*

Analoges Vorgehen wie in Beispiel 2 mit n-1-Jodperfluorheptan anstellen von Pentafluorethyljodid liefert das gewünschte Halbaminal als gelbes Öl in 43% Ausbeute.

Sdp. 80°C/0,0165 mbar; $^1$H-NMR (300 MHz, $CDCl_3$): 2,41 ppm, s, 6H, 2 $CH_3$-N; 4,60 ppm, t, JHF = 11,25 Hz, 1 H-C(8).

*Anwendungsbeispiel*

*Beispiel 10*

*2,2-Dichlor-3,3,3-trifluorpropanal*

In einer Apparatur bestehend aus einem 250 ml Dreihalsrundkolben mit $N_2$-Einlass, einem Tropfrichter und 2 nachgeschalteten Kühlfallen (−78°C) werden 49,0 g (0,5 Mol) konzentriertes $H_2SO_4$ vorgelegt und auf 90°C erhitzt. Nun tropft man langsam 149,0 g (0,5 Mol) 1,1,1-Trifluor-2,2-dichlor-3-dimethylamino-3-trimethylsilyloxypropan ein und kondensiert den im $N_2$-Strom übergeleiteten Aldehyd. Bei der Redestillation geht der Aldehyd in 83% Ausbeute (75,0 g) als farbloses Öl bei 52°C/994 mbar über.

$^1$H-NMR (60 MHz, $CDCl_3$): 9,30 ppm, qa, JHF = 2,5 Hz, C*H*O.

**Patentansprüche**

1. Verfahren zur Herstellung von Halbaminalen der Formel I

```
     R1
     |
R2-SiO-CH-N(CH3)2          (I),
     |   |
     R3  R
```

worin R unsubstituiertes oder mit -$NO_2$, -CN, -COR', -COOR, -CON(R')$_2$, $C_1$-$C_{12}$-Alkyl, $C_1$-$C_{12}$-Alkoxy, $C_1$-$C_{12}$-Alkylthio, $C_1$-$C_{12}$-Sulfoxyl, $C_1$-$C_{12}$-Sulfonyl, Phenyl, Benzyl, $C_3$-$C_{18}$-Trialkylsilyl oder -silyloxy und $C_1$-$C_{18}$-Mono- oder Dialkylamino, substituertes polyhalogeniertes Alkyl, Cycloalkyl oder Aralkyl mit jeweils mindestens einem F-Atom bedeutet, wobei R' ein Wasserstoffatom, $C_1$-$C_{18}$-Alkyl, $C_4$-$C_8$-Cycloalkyl, Phenyl oder Benzyl bedeutet, oder die Arylgruppe von R als Aralkyl auch mit -OH substituiert sein kann und $R^1$, $R^2$ und $R^3$ unabhängig voneinander ein Wasserstoffatom, Alkyl, Cycloalkyl oder Aryl darstellen, das dadurch gekennzeichnet ist, dass man

a) mindestens äquimolare Mengen Dimethylformamid, eines Silans der Formel II

$$R^1R^2R^3SiX \qquad (II),$$

worin X Cl oder Br ist, und $R^1$, $R^2$, $R^3$ die zuvor angegebene Bedeutung haben, und einer Verbindung der Formel III

$$RY \qquad (III),$$

worin R die zuvor angegebene Bedeutung hat und Y für Cl, Br oder J steht, in Gegenwart von Zink miteinander umsetzt, oder

b) mindestens äquimolare Mengen Dimethylformamid, eines Silans der Formel II und einer Zinkverbindung der Formel IV

$$RZnY \cdot yL \qquad (IV),$$

worin R und Y die zuvor angegebene Bedeutung haben, L einen Liganden bedeutet und y 1 oder 2 ist, miteinander umsetzt.

2. Verfahren gemäss Anspruch 1, dadurch gekennzeichnet, dass die Reaktion a) bei einer Temperatur von −30°C bis 140°C und die Reaktion b) bei einer Temperatur von 20°C bis 200°C durchgeführt wird.

3. Verfahren gemäss Anspruch 1, dadurch gekennzeichnet, dass X in Formel II für Cl steht.

4. Verfahren gemäss Anspruch 1, dadurch gekennzeichnet, dass man bei der Reaktion b) zunächst die Zinkverbindung der Formel IV erzeugt durch die Umsetzung von einer Verbindung der Formel III mit Zink in Gegenwart eines Lösungsmittels, das dem Liganden L entspricht.

5. Verfahren gemäss Anspruch 1, dadurch gekennzeichnet, dass L in Formel IV Dimethylformamid und y = 2 bedeuten, und die Reaktion b) in Gegenwart eines inerten Lösungsmittels durchgeführt wird.

6. Verfahren gemäss Anspruch 1, dadurch gekennzeichnet, dass L in Formel IV ein Heteroatome enthaltendes aprotisches Lösungsmittel bedeutet.

7. Verfahren gemäss Anspruch 6, dadurch gekennzeichnet, dass L in Formel IV ausgewählt ist aus der Gruppe der Ether, der Carbonsäureester und Lactone, der Sulfoxide und Sulfone und der N-substituierten Säureamide und Lactame.

8. Verfahren gemäss Anspruch 1, dadurch gekennzeichnet, dass R in Formel I ein bis drei Substituenten enthält.

9. Verfahren gemäss Anspruch 1, dadurch gekennzeichnet, dass $R^1$, $R^2$ und $R^3$ gleiches oder verschiedenes $C_1$-$C_8$-Alkyl bedeuten.

10. Verfahren gemäss Anspruch 1, dadurch gekennzeichnet, dass R in Formel I mit F polyhalogeniert ist.

11. Verfahren gemäss Anspruch 1, dadurch gekennzeichnet, dass R in Formel I gegebenenfalls sub-

stituiertes, polyhalogeniertes $C_1$-$C_{18}$-Alkyl, polyhalogeniertes Cycloalkyl mit 3 bis 8 C-Atomen im Ring, oder polyhalogeniertes Benzyl bedeutet.

12. Halbaminale der Formel I

$R^1$
|
$R^2$-SiO-CH-N($CH_3$)$_2$ (I),
| |
$R^3$ R

worin R unsubstituiertes oder mit -$NO_2$, -CN, -COR', -COOR', -CON(R')$_2$, $C_1$-$C_{12}$Alkyl, $C_1$-$C_{12}$-Alkoxy, $C_1$-$C_{12}$Alkylthio, $C_1$-$C_{12}$-Sulfoxyl, $C_1$-$C_{12}$-Sulfonyl, Phenyl, Benzyl, $C_3$-$C_{18}$-Trialkylsilyl oder -silyloxy und $C_1$-$C_{18}$-Mono- oder Dialkylamino, substituiertes polyhalogeniertes Alkyl, Cycloalkyl oder Aralkyl mit jeweils mindestens einem F-Atom bedeutet, wobei R' ein Wasserstoffatom, $C_1$-$C_{18}$-Alkyl, $C_4$-$C_8$-Cycloalkyl, Phenyl oder Benzyl bedeutet, oder die Arylgruppe von R als Aralkyl auch mit -OH substituiert sein kann und $R^1$, $R^2$ und $R^3$ unabhängig voneinander ein Wasserstoffatom, Alkyl, Cycloalkyl oder Aryl darstellen.

13. Verwendung von Halbaminalen der Formel I gemäss Anspruch 1 zur Herstellung von Aldehyden der Formel R-CHO durch Hydrolyse mit Mineralsäuren.

**Claims**

1. A process for the preparation of a hemiaminal of formula I

$R^1$
|
$R^2$-SiO-CH-N($CH_3$)$_2$ (I)
| |
$R^3$ R

in which R is polyhalogenated alkyl, cycloalkyl or aralkyl which each have at least one F atom and are unsubstituted or substituted by -$NO_2$, -CN, -COR', -COOR', -CON(R')$_2$, $C_1$-$C_{12}$alkyl, $C_1$-$C_{12}$alkoxy, $C_1$-$C_{12}$alkylthio, $C_1$-$C_{12}$sulfoxyl, $C_1$-$C_{12}$sulfonyl, phenyl, benzyl, $C_3$-$C_{18}$trialkylsilyl or -silyloxy and $C_1$-$C_{18}$monoalkylamino or $C_1$-$C_{18}$dialkylamino, R' being a hydrogen atom, $C_1$-$C_{18}$alkyl, $C_4$-$C_8$-cycloalkyl, phenyl or benzyl, or the aryl group of R as aralkyl may also be substituted by -OH and $R^1$, $R^2$ and $R^3$ independently of one another are a hydrogen atom, alkyl, cycloalkyl or aryl, which comprises

a) reacting with one another at least equimolar amounts of dimethylformamide, a silane of formula II

$R^1R^2R^3SiX$ (II)

in which X is Cl or Br, and $R^1$, $R^2$ and $R^3$ are as defined above, and a compound of formula III

RY (III)

in which R is as defined above and Y is Cl, Br or I, in the presence of zinc, or

b) reacting with one another at least equimolar amounts of dimethylformamide, a silane of formula II and a zinc compound of formula IV

RZnY·yL (IV)

in which R and Y are as defined above, L is a ligand and y is 1 or 2.

2. A process according to claim 1, wherein reaction a) is carried out at a temperature from −30°C to 140°C and reation b) is carried out at a temperature from 20°C to 200°C.

3. A process according to claim 1, wherein X in formula II is Cl.

4. A process according to claim 1, wherein in reaction b) the zinc compound of formula IV is first produced by reacting a compound of formula III with zinc in the presence of a solvent corresponding to the ligand L.

5. A process according to claim 1, wherein L in formula IV is dimethylformamide and y = 2 and reaction b) is carried out in the presence of an inert solvent.

6. A process according to claim 1, wherein L in formula IV is an aprotic solvent containing hetero atoms.

7. A process according to claim 6, wherein L in formula IV is selected from the group consisting of ethers, carboxylic esters and lactones, sulfoxides and sulfones and N-substituted acid amides and lactams.

8. A process according to claim 1, wherein R in formula I contains one to three substituents.

9. A process according to claim 1, wherein $R^1$, $R^2$ and $R^3$ are identical or different $C_1$-$C_8$alkyl radicals.

10. A process according to claim 1, wherein R in formula I is polyhalogenated by F.

11. A process according to claim 1, wherein R in formula I is unsubstituted or substituted polyhalogenated $C_1$-$C_{18}$alkyl, polyhalogenated cycloalkyl having 3 to 8 C atoms in the ring, or is polyhalogenated benzyl.

12. A hemiaminal of formula I

$R^1$
|
$R^2$-SiO-CH-N($CH_3$)$_2$ (I),
| |
$R^3$ R

in which R is polyhalogenated alkyl, cycloalkyl or aralkyl which each have at least one F atom and are unsubstituted or substituted by -$NO_2$, -CN, -COR', -COOR', -CON(R')$_2$, $C_1$-$C_{12}$alkyl, $C_1$-$C_{12}$alkoxy, $C_1$-$C_{12}$alkylthio, $C_1$-$C_{12}$sulfoxyl, $C_1$-$C_{12}$sulfonyl, phenyl, benzyl, $C_3$-$C_{18}$trialkylsilyl or -silyloxy and $C_1$-$C_{18}$monoalkylamino or $C_1$-$C_{18}$dialkylamino, R' being a hydrogen atom, $C_1$-$C_{18}$alkyl, $C_4$-$C_8$-cycloalkyl, phenyl or benzyl, or the aryl group of R as aralkyl may also be substituted by -OH and $R^1$, $R^2$ and $R^3$ independently of one another are a hydrogen atom, alkyl, cycloalkyl or aryl.

13. Use fo a hemiaminal of formula I according to claim 1 for preparing an aldehyde of formula R-CHO by hydrolysis with a mineral acid.

## Revendications

1. Procédé pour préparer des hémi-aminals répondant à la formule I:

$$\begin{array}{l} R^1 \\ | \\ R^2\text{-SiO-CH-N}(CH_3)_2 \qquad (I), \\ \;|\quad\;\; | \\ R^3\;\; R \end{array}$$

dans laquelle R représente un radical alkyle, cycloalkyle ou aralkyle polyhalogéné, au moins monofluoré, non substitué ou porteur d'un substituant pris dans l'ensemble constitué par $-NO_2$, -CN, -COR', -COOR', $-CON(R')_2$, les alkyles en $C_1$-$C_{12}$, les alcoxy en $C_1$-$C_{12}$, les alkylthio en $C_1$-$C_{12}$, les sulfinyles en $C_1$-$C_{12}$, les sulfonyles en $C_1$-$C_{12}$ les phényle, le benzyle, les trialkylsilyles en $C_3$-$C_{18}$, les trialkylsilyloxy en $C_3$-$C_{18}$ et les mono- et dialkylamino à alkyle en $C_1$-$C_{18}$, le symbole R' représentant un atome d'hydrogène, un alkyle en $C_1$-$C_{18}$, un cycloalkyle en $C_4$-$C_8$, un phényle ou un benzyle, et le radical aryle de l'aralkyle R pouvant également porter un radical -OH, et $R^1$, $R^2$ et $R^3$ représentent chacun, indépendamment les uns des autres, un atome d'hydrogène, un alkyle, un cycloalkyle ou un aryle, procédé caractérisé en ce que:

a) on fait réagir ensemble, en présence de zinc, des quantités au moins équimolaires de diméthylformamide, d'un silane répondant à la formule II:

$$R^1R^2R^3SiX \qquad (II)$$

dans laquelle X représente Cl ou Br, et $R^1$, $R^2$ et $R^3$ ont les significations qui leur ont été données ci-dessus, et d'un composé répondant à la formule III:

$$RY \qquad (III)$$

dans laquelle R a la signification qui lui a été donnée ci-dessus, et Y représente Cl, Br ou I, ou

b) on fait réagir ensemble des quantités au moins équimolaires de diméthylformamide, d'un silane de formule II et d'un composé du zinc répondant à la formule IV:

$$RZnY \cdot yL \qquad (IV)$$

dans laquelle R et Y ont les significations qui leur ont été données ci-dessus, L représente un coordinat, et y est égal à 1 ou à 2.

2. Procédé selon la revendication 1 caractérisé en ce que la réaction a) est effectuée à une température de −30 à 140°C, et la réaction b) à une température de 20 à 200°C.

3. Procédé selon la revendication 1 caractérisé en ce que X, dans la formule II, représente le chlore.

4. Procédé selon la revendication 1 caractérisé en ce que, dans la réaction b), on crée d'abord le composé du zinc de formule IV par réaction d'un composé de formule III avec le zinc en présence d'un solvant correspondant au coordinat L.

5. Procédé selon la revendication 1 caractérisé en ce que, dans la formule IV, L représente le diméthylformamide et y est égal à 2, et la réaction b) est effectuée en présence d'un solvant inerte.

6. Procédé selon la revendication 1 caractérisé en ce que, dans la formule IV, L représente un solvant aprotique contenant des hétéro-atomes.

7. Procédé selon la revendication 6 caractérisé en ce que, dans la formule IV, L est choisi dans l'ensemble constitué par les éthers, les esters d'acides carboxyliques, les lactones, les sulfoxydes, les sulfones, les amides substitués à l'azote et les lactames.

8. Procédé selon la revendication 1 caractérisé en ce que, dans la formule I, le radical R porte de 1 à 3 substituants.

9. Procédé selon la revendication 1 caractérisé en ce que $R^1$, $R^2$ et $R^3$ représentent des radicaux alkyles en $C_1$-$C_8$, identiques ou différents.

10. Procédé selon la revendication 1 caractérisé en ce que, dans la formule I, le radical R est polyhalogéné par F.

11. Procédé selon la revendication 1 caractérisé en ce que, dans la formule I, R représente un radical alkyle en $C_1$-$C_{18}$ polyhalogéné, éventuellement substitué, un radical cycloalkyle polyhalogéné contenant de 3 à 8 atomes de carbone dans son cycle, ou un radical benzyle polyhalogéné.

12. Hémi-aminals répondant à la formule I:

$$\begin{array}{l} R^1 \\ | \\ R^2\text{-SiO-CH-N}(CH_3)_2 \qquad (I), \\ \;|\quad\;\; | \\ R^3\;\; R \end{array}$$

dans laquelle R représente un radical alkyle, cycloalkyle ou aralkyle polyhalogéné, au moins fluoré, non substitué porteur d'un substituant pris dans l'ensemble constitué par $-NO_2$, -CN, -COR', -COOR', $-CON(R')_2$, les alkyles en $C_1$-$C_{12}$, les alcoxy en $C_1$-$C_{12}$, les alkylthio en $C_1$-$C_{12}$, les sulfinyles en $C_1$-$C_{12}$, les sulfonyles en $C_1$-$C_{12}$, les phényle, le benzyle, les trialkylsilyles en $C_3$-$C_{18}$, les trialkylsilyloxy en $C_3$-$C_{18}$ et les mono- et dialkylamino à alkyle en $C_1$-$C_{18}$, le symbole R' représentant un atome d'hydrogène, un alkyle en $C_1$-$C_{18}$, un cycloalkyle en $C_4$-$C_8$, un phényle ou un benzyle, et le radical aryle de l'aralkyle R pouvant également porter un radical -OH, et $R^1$, $R^2$ et $R^3$ représentent chacun, indépendamment les uns des autres, un atome d'hydrogène, un alkyle, un cycloalkyle ou un aryle.

13. Application d'hémi-aminals de formule I selon la revendication 1 à la préparation d'aldéhydes de formule R-CHO par hydrolyse avec des acides minéraux.